Europäisches Patentamt

European Patent Office

Office européen des brevets

(11) Veröffentlichungsnummer: **0 360 986 B1**

# EUROPÄISCHE PATENTSCHRIFT

(45) Veröffentlichungstag der Patentschrift: **19.11.92**

(51) Int. Cl.⁵: **A61K 7/09**

(21) Anmeldenummer: **89111640.2**

(22) Anmeldetag: **27.06.89**

(54) Fixiermittel und Verfahren zur dauerhaften Haarverformung.

(30) Priorität: **16.07.88 DE 3824241**

(43) Veröffentlichungstag der Anmeldung:
**04.04.90 Patentblatt 90/14**

(45) Bekanntmachung des Hinweises auf die
Patenterteilung:
**19.11.92 Patentblatt 92/47**

(84) Benannte Vertragsstaaten:
**AT BE CH DE ES FR GB GR IT LI NL SE**

(56) Entgegenhaltungen:
DE-A- 1 467 853          DE-A- 1 617 829
DE-A- 2 624 690          DE-A- 3 814 356
DE-A- 3 814 685          FR-A- 2 051 629

CHEMICAL ABSTRACTS, Band 85, Nr. 16, 18.
Oktober 1976, Seite 267, Spalte 1, Zusammenfassung Nr. 112653b, Columbus, Ohio,
USA

CHEMICAL ABSTRACTS, Band 69, Nr. 16, 14.
Oktober 1968, Seite 5762, Spalte 1, Zusammenfassung Nr. 61495m, Columbus, Ohio,
USA; A. SHANSKY: "Methods and compositions for bleaching and lightening human
hair"

(73) Patentinhaber: **Wella Aktiengesellschaft
Berliner Allee 65
W-6100 Darmstadt(DE)**

(72) Erfinder: **Clausen, Thomas, Dr.
Ernst-Pasqué-Strasse 35 a
W-6146 Alsbach(DE)**
Erfinder: **Hoch, Dietrich
Riedstrasse 30
W-6102 Pfungstadt-Eich(DE)**
Erfinder: **Lang, Günther, Dr.
Auf der roten Erde 10 b
W-6107 Reinheim 5(DE)**

EP 0 360 986 B1

**Beschreibung**

Die vorliegende Erfindung betrifft ein saures Fixiermittel für die Durchführung der oxidativen Nachbehandlung bei der dauerhaften Haarverformung, das eine Mischung aus Wasserstoffperoxid und einem physiologisch verträglichen Oxidationsmittel, dessen Oxidationspotential größer ist als das Oxidationspotential von Wasserstoffperoxid, enthält, sowie ein Verfahren zur dauerhaften Haarverformung unter Verwendung dieses Fixiermittels.

Bei der dauerhaften Haarverformung wird das Haar zunächst mit einem Verformungsmittel, welches eine Öffnung der S-S-Bindungen des Haarkeratins bewirkt, behandelt und sodann in die gewünschte Form gebracht. Als Verformungsmittel werden hierbei in der Regel keratinreduzierende Mercaptoverbindungen, wie zum Beispiel Salze oder Ester von Mercaptocarbonsäuren, verwendet. Anschließend wird das Haar mit Wasser gespült und sodann mit einem Fixiermittel oxidativ nachbehandelt. Hierbei werden die S-S-Bindungen des Haarkeratins wiederhergestellt. Da bei der dauerhaften Haarverformung weder die keratinreduzierende noch die oxidative Stufe vollständig abläuft, verbleiben auch bei sorgfältiger Arbeitsweise geringe Mengen an nicht oxidierten Mercaptangruppen im Haar. Hierdurch sowie durch im Haar verbleibende Reste des Verformungsmittels erhält das Haar einen unangenehmen Geruch, der auch durch mehrmalige Haarwäsche nicht vollständig entfernt werden kann. Insbesondere bei Verwendung von Verformungsmitteln auf der Basis von Mercaptocarbonsäureestern tritt eine derartige Geruchsbelästigung häufig auf.

Es wurden bereits verschiedene Vorschläge gemacht, den unangenehmen Mercaptangeruch der Haare zü beseitigen. So wurde beispielsweise vorgeschlagen, den Verformungsmitteln Parfümöle welche den Mercaptangeruch überdecken sollen, zuzusetzen. Ebenfalls ist es aus der DE-OS 34 16 075 bekannt, Verformungsmitteln zur Beseitigung des Mercaptangeruches eine Kombination aus bestimmten zyklischen, Carbonylgruppen enthaltenden, Verbindungen und alpha-, beta- gamma- oder delta-Cyclodextrinen zuzusetzen. Mit solchen Zusätzen läßt sich zwar der unangenehme Geruch der Verformungsmittel teilweise beseitigen, das Problem der Geruchsbelastung der Haare nach einer Dauerverformungsbehandlung bleibt jedoch unverändert bestehen.

In der DE-OS 34 33 648 wird vorgeschlagen, zur Beseitigung des Mercaptangeruches für die oxidative Nachbehandlung der Haare ein Fixiermittel zu verwenden, das eine Kombination eines üblichen Oxidationsmittels - beispielsweise Wasserstoffperoxid, Natriumperborat, Natriumpercarbonat oder Alkalibromate sowie Mischungen dieser Verbindungen - mit bestimmten Carbonylverbindungen enthält. Ein Fixiermittel, das eine Kombination von Wasserstoffperoxid mit einem Oxidationsmittel, dessen Oxidationspotential größer ist als das Oxidationspotential von Wasserstoffperoxid, enthält, wird jedoch in der DE-OS 34 33 648 nicht beschrieben. Außerdem ist die Verwendung eines Teils der in der DE-OS 34 33 648 als Zusatz empfohlenen Carbonylverbindungen physiologisch nicht völlig unbedenklich.

Es bestand daher die Aufgabe, den bei der dauerhaften Haarverformung auftretenden und dem Haar anhaftenden unangenehmen Mercaptangeruch zu beseitigen, ohne hierdurch die Haarstruktur zu beeinträchtigen oder die physiologische Verträglichkeit des Fixiermittels zu verschlechtern.

Überraschenderweise wurde nunmehr gefunden, daß die gestellte Aufgabe in hervorragender Weise gelöst wird, wenn bei der dauerhaften Haarverformung für die Durchführung der oxidativen Nachbehandlung ein Fixiermittel verwendet wird, das eine Kombination aus Wasserstoffperoxid und einem weiteren physiologisch verträglichen Oxidationsmittel, dessen Oxidationspotential größer ist als das Oxidationspotential von Wasserstoffperoxid, enthält.

Gegenstand der vorliegenden Erfindung ist somit ein saures Fixiermittel für die Durchführung der oxidativen Nachbehandlung bei der dauerhaften Haarverformung auf der Basis von Wasserstoffperoxid, welches dadurch gekennzeichnet ist, daß es ein weiteres physiologisch verträgliches Oxidationsmittel, dessen Oxidationspotential größer ist als das Oxidationspotential von Wasserstoffperoxid, enthält.

Als physiologisch verträgliches Oxidationsmittel, dessen Oxidationspotential größer ist als das Oxidationspotential von Wasserstoffperoxid, wird vorzugsweise ein Alkali- oder Ammoniumperoxodisulfat verwendet.

Das erfindungsgemäße Fixiermittel enthält vorzugsweise 0,1 bis 18 Gewichtsprozent Wasserstoffperoxid und 0,05 bis 5 Gewichtsprozent eines physiologisch verträglichen Oxidationsmittels, dessen Oxidationspotential größer ist als das Oxidationspotential von Wasserstoffperoxid.

Besonders bevorzugt sind solche Fixiermittel, die 1,5 bis 12 Gewichtsprozent Wasserstoffperoxid und 0,1 bis 4 Gewichtsprozent eines Alkali- oder Ammoniumperoxodisulfates enthalten.

Das molare Verhältnis von Wasserstoffperoxid zu dem physiologisch verträglichen Oxidationsmittel, dessen Oxidationspotential größer ist als das Oxidationspotential von Wasserstoffperoxid, beträgt in dem erfindungsgemäßen Fixiermittel vorzugsweise etwa 1 : 0,0076 bis 1 : 0,76, wobei ein molares Verhältnis von 1 : 0,01 bis 1 : 0,23 besonders bevorzugt ist.

EP 0 360 986 B1

Dem Fixiermittel können übliche Quell- und Penetrationsstoffe, wie zum Beispiel Harnstoff, 2-Pyrrolidon, 1-Methyl-2-pyrrolidon und Dipropylenglykolmonomethylether, sowie Peroxidstabilisatoren, beispielsweise aromatische Sulfonsäuren, Salzsäure, Schwefelsäure, Phosphorsäure, Pyro- oder Polyphosphorsäuren, saure Salze starker Säuren, Ascorbinsäure, Oxalsäure, Malonsäure, Benzoesäure, Salicylsäure, Zitronensäure, Gerbsäuren, Paraformaldehyd, 4-Acetamido-phenol, Phenol, Thymol oder alpha-Bisabolol, zugesetzt werden.

Weiterhin können in dem Fixiermittel Netzmittel und Emulgatoren aus der Gruppe der anionischen, nichtionischen, kationischen und amphoteren oder zwitterionischen oberflächenaktiven Agenzien enthalten sein, unter der Voraussetzung, daß der hydrophobe Anteil dieser Agenzien gesättigt und nicht oxidierbar ist. Geeignete oberflächenaktive Agenzien sind insbesondere

a) anionische oberflächenaktive Agenzien, wie beispielsweise Alkali-, Erdalkali-, Ammonium- oder Alkanolaminsalze von Alkylsulfonaten, Alkylsulfaten und Alkylethersulfaten, wie zum Beispiel Natriumlaurylalkoholdiglykolethersulfat, Natrium- oder Triethanolaminsalze von Alkylsulfaten mit 12 bis 18, vorzugsweise 12 bis 14 Kohlenstoffatomen, die Natrium- oder Triethanolaminsalze von Lauryl- oder Tetradecylethersulfaten, das Dinatriumsalz des Sulfosuccinhalbesters von Alkanolamiden, Seifen und Polyethercarbonsäuren;

b) nichtionische oberflächenaktive Agenzien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl- und Stearylalkohol, allein oder im Gemisch, oxethylierte Lanolinalkohole, oxethyliertes Lanolin, oxethylierte Alkylphenole mit 8 bis 30 Kohlenstoffatomen im Alkylrest und 1 bis 10 Ethylenoxideinheiten im Molekül, Fettsäurealkanolamide sowie oxethylierte Sorbitanfettsäureester;

c) kationische oberflächenaktive Agenzien, wie beispielsweise Dilauryldimethylammoniumchlorid, Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder -bromide, Dialkyldimethylammoniumchloride oder -bromide, Alkylpyridiniumsalze, beispielsweise Lauryl- oder Cetylpyridiniumchlorid, Alkylamidoethyltrimethylammoniumethersulfate, Verbindungen mit kationischem Charakter wie Aminoxide, beispielsweise Alkyldimethylaminoxide oder Alkylaminoethyldimethylaminoxide und

d) amphotere oder zwitterionische oberflächenaktive Agenzien wie beispielsweise Carboxylderivate des Imidazols, N-Alkyl und N-Alkylamidobetaine, N-Alkylsulfobetaine, N-Alkylaminopropionate, Alkyldimethylcarboxymethylammoniumsalze mit 12 bis 18 Kohlenstoffatomen sowie Fettsäurealkylamidobetaine, beispielsweise Fettsäureamidopropyldimethylaminoessigsäurebetain.

Selbstverständlich kann das Fixiermittel alle für derartige Mittel üblichen Zusatzstoffe, zum Beispiel Verdickungsmittel, wie beispielsweise Kaolin, Bentonit, Fettsäuren, höhere Fettalkohole, Stärke, Polyacrylsäure, Cellulosederivate, Alginate, Vaseline oder Paraffinöl, ferner Farbstoffe, Trübungsmittel, wie zum Beispiel Polyethylenglykolester, oder Alkohole, wie beispielsweise Ethanol, Propanol und Isopropanol, Lösungsvermittler, Puffersubstanzen, Parfümöle, haarkonditionierende oder haarpflegende Bestandteile, wie zum Beispiel Lanolinderivate, Cholesterin oder Betain, enthalten.

Die genannten Zusatzstoffe werden in den für solche Zwecke üblichen Mengen verwendet, beispielsweise die Verdickungsmittel in einer Menge von etwa 0,1 bis 25 Gewichtsprozent.

Das Fixiermittel liegt in Form einer wäßrigen oder wäßrig-alkoholischen Lösung oder Emulsion vor und besitzt einen pH-Wert von 1,5 bis 6,9, vorzugsweise von 1,5 bis 3,5. Es kann jedoch auch in verdickter Form auf wäßriger Basis, insbesondere als Creme, Gel oder Paste, vorliegen.

Vorzugsweise liegt das Fixiermittel in Form eines Zweikomponentenpräparates vor und wird erst unmittelbar vor dem Gebrauch durch Vermischen der wasserfreien, das physiologisch verträgliche Oxidationsmittel, dessen Oxidationspotential größer ist als das Oxidationspotential von Wasserstoffperoxid, enthaltenden Komponente mit der wäßrigen, Wasserstoffperoxid enthaltenden Komponente hergestellt. Die vorstehend aufgeführten Zusatzstoffe können hierbei, je nach ihren Eigenschaften, sowohl in der wäßrigen als auch in der wasserfreien Komponente enthalten sein.

Bei Verwendung des vorstehend beschriebenen Fixiermittels für die oxidative Nachbehandlung bei der dauerhaften Haarverformung wird eine gute Fixierung des Haares bei gleichzeitiger Beseitigung des unangenehmen Geruches erreicht, ohne hierdurch die Haarstruktur negativ zu beeinflussen.

Ein weiterer Gegenstand der vorliegenden Erfindung ist daher ein Verfahren zur dauerhaften Haarverformung, bei dem man das Haar bevor und/oder nachdem man es in die gewünschte Form bringt mit einem keratinreduzierenden Verformungsmittel behandelt, mit Wässer spült, sodann mit einem Fixiermittel oxidativ nachbehandelt, mit Wasser spült, anschließend zur Frisur legt und sodann trocknet, welches dadurch gekennzeichnet ist, daß für die oxidative Nachbehandlung das vorstehend beschriebene erfindungsgemäße Fixiermittel verwendet wird.

3

Bei dem erfindungsgemäßen Verfahren wird das Haar gewaschen, mit einem Handtuch frottiert, gegebenenfalls mit einem Teil des keratinreduzierenden Verformungsmittels vorgefeuchtet, in einzelne Strähnen aufgeteilt und auf Wickler gewickelt. Der Durchmesser der Wickler beträgt hierbei, je nachdem ob ein Dauerwellung oder eine Entkräuselung der Haare gewünscht wird, entweder etwa 5 bis 13 Millimeter oder etwa 15 bis 35 Millimeter. Auf das gewickelte Haar wird anschließend eine für die dauerhafte Haarverformung ausreichende Menge des Verformungsmittels aufgetragen. Die für die dauerhafte Haarverformung erforderliche Gesamtmenge des Verformungsmittels beträgt im allgemeinen etwa 80 bis 120 Gramm.

Die bei dem erfindungsgemäßen Verfahren verwendbaren Verformungsmittel enthalten übliche keratinreduzierende Verbindungen, wie zum Beispiel bestimmte Mercaptoverbindungen, insbesondere Thioglycerin, Cysteamin sowie Salze oder Ester von Mercaptocarbonsäuren. Diese Verformungsmittel enthalten die keratinreduzierenden Verbindungen in den für derartige Mittel üblichen Mengen, beispielsweise die Ammoniumsalze der Thioglykol- oder Thiomilchsäure in einer Menge von etwa 2 bis 12 Gewichtsprozent. Der pH-Wert dieser Verformungsmittel beträgt im allgemeinen etwa 7 bis 11, wobei die Einstellung des pH-Wertes vorzugsweise mit Ammoniak, Monoethanolamin, Ammoniumcarbonat oder Ammoniumhydrogencarbonat erfolgt. Bei sauer (zum Beispiel auf pH = 6,5 bis 6,9) eingestellten Verformungsmitteln werden vorzugsweise Ester von Mercaptocarbonsäuren, wie beispielsweise Monothioglykolsäureglykolester-oder -glycerinester, in einer Konzentration von etwa 2 bis 25 Gewichtsprozent verwendet.

Die Verformungsmittel können weiterhin alle für derartige Mittel üblichen Zusatzstoffe, beispielsweise Quell- und Penetrationsstoffe, Verdickungsmittel, Netzmittel und Emulgatoren, Alkohole, Lösungsvermittler, Stabilisatoren, Farbstoffe, Parfümöle sowie haarkonditionierende oder haarpflegende Bestandteile, enthalten. Die vorstehend genannten Zusatzstoffe werden in den für solche Zwecke üblichen Mengen verwendet, zum Beispiel die Netzmittel und Emulgatoren in Konzentrationen von etwa 0,2 bis 30 Gewichtsprozent, während die Verdickungsmittel in einer Menge von etwa 0,1 bis 25 Gewichtsprozent in dem Verformungsmittel enthalten sein können

Das bei dem erfindungsgemäßen Verfahren verwendete Verformungsmittel kann sowohl in Form einer wäßrigen Lösung oder Emulsion als auch in verdickter Form auf wäßriger Basis, insbesondere als Creme, Gel oder Paste, oder in Form eines Aerosolschaums vorliegen.

Nach einer für die dauerhafte Haarverformung ausreichenden Einwirkungszeit, welche je nach der Haarbeschaffenheit, dem pH-Wert und der Verformungswirksamkeit des Verformungsmittels sowie in Abhängigkeit von der Anwendungstemperatur etwa 5 bis 45 Minuten (5 bis 30 Minuten mit Wärmeeinwirkung; 20 bis 45 Minuten ohne Wärmeeinwirkung) beträgt, wird das Haar mit Wasser gespült und sodann mit etwa 80 bis 120 Gramm des vorstehend beschriebenen erfindungsgemäßen Fixiermittels oxidativ nachbehandelt.

Nach einer Einwirkungszeit von etwa 1 bis 20 Minuten werden die Wickler entfernt und das abgewickelte Haar, falls erforderliche, nochmals mit dem Fixiermittel oxidativ nachbehandelt. Sodann wird das Haar mit Wasser gespült, zur Frisur gelegt und getrocknet.

Das so behandelte Haar besitzt eine gleichmäßige und haltbare Umformung und ist frei von unangenehmem Mercaptangeruch.

Die nachfolgenden Beispiele sollen den Gegenstand der vorliegenden Erfindung näher erläutern, ohne den Gegenstand auf diese Beipiele zu beschränken.

**Beispiele**

**Beispiel 1: Fixiermittel**

| Komponente 1: | 1,50 g | Wasserstoffperoxid |
|---|---|---|
| | 3,75 g | Natriumlaurylalkoholdiglykolethersulfat |
| | 0,50 g | Zitronensäure |
| | 0,50 g | Natriumdihydrogenphosphat |
| | 92,65 g | Wasser |
| | 98,90 g | |
| Komponente 2: | 0,10 g | Ammoniumperoxodisulfat |
| | 1,00 g | Natriumsulfat |
| | 1,10 g | |
| Komponente 1 + 2: | 100,00 g | |

Komponente 1 und Komponente 2 werden unmittelbar vor Gebrauch miteinander vermischt. Der pH-Wert der gebrauchsfertigen Zubereitung beträgt 2,9.

Im Anschluß an die Behandlung der Haare mit einem Verformungsmittel auf Thioglykolatbasis wird das Haar mit Wasser gespült und sodann das vorstehend beschriebene Fixiermittel mit einem Schwamm aufgetragen. Nach einer Einwirkungszeit von 10 Minuten werden die Wickler entfernt und das Haar mit lauwarmem Wasser ausgespült. Anschließend wird das Haar zur Frisur gelegt und sodann getrocknet.

Das so behandelte Haar ist frei von störendem Mercaptangeruch.

**Beispiel 2: Fixiermittel**

| Komponente 1: | 3,0 g | Wasserstoffperoxid |
|---|---|---|
| | 2,0 g | Cetylstearylalkohol |
| | 1,0 g | Wollwachs (Adeps Lanae) |
| | 1,0 g | Nonylphenol, mit 10 Mol Ethylenoxid oxethyliert |
| | 0,5 g | alpha-Bisabolol |
| | 0,5 g | 2-Octyl-decanol |
| | 0,5 g | Natriumlaurylsulfat |
| | 0,2 g | Parfümöl |
| | 0,1 g | ortho-Phosphorsäure, 85-prozentige wäßrige Lösung |
| | 87,2 g | Wasser |
| | 96,0 g | |
| Komponente 2: | 4,0 g | Ammoniumperoxodisulfat |
| Komponente 1 + 2: | 100,0 g | |

Unmittelbar vor dem Gebrauch werden die Komponente 1 und die Komponente 2 zu einem Fixiermittel mit pH 2,0 vermischt.

Die Anwendung dieses Fixiermittels erfolgt in der in Beispiel 1 beschriebenen Weise.

Die so behandelten Haare besitzen einen neutralen Geruch und sind frei von unangenehmem Mercaptangeruch.

**Beispiel 3: Fixiermittel**

| Komponente 1: | 2,0 g | Wasserstoffperoxid |
| | 1,0 g | Dimethylaminoethylmethacrylat, zu 75 % mit Dimethylsulfat quaternisiert |
| | 0,6 g | Fettsäureamidopropyldimethylaminoessigsäurebetain |
| | 0,5 g | Lauryldimethylaminoxid |
| | 0,2 g | Ethylendiaminotetraessigsäure |
| | 0,2 g | Natriumdihydrogenphosphat |
| | 0,1 g | Phenacetin |
| | 92,4 g | Wasser |
| | 97,0 g | |
| Komponente 2: | 3,0 g | Natriumperoxodisulfat |
| Komponente 1 + 2: | 100,0 g | |

Komponente 1 und 2 werden unmittelbar vor Gebrauch zu einem Fixiermittel mit pH 2,8 vermischt.

Im Anschluß an eine Haarwäsche wird das handtuchtrockene Haar auf Wickler mit einem Durchmesser von 10 Millimetern gewickelt und sodann mit 100 g einer 10-prozentigen wäßrigen Ammoniumthioglykolatlösung, deren pH-Wert mit Ammoniumcarbonat/Ammoniumhydrogencarbonat auf 9,0 eingestellt ist, gleichmäßig durchfeuchtet. Nach einer Einwirkungszeit von 20 Minuten bei Raumtemperatur wird das Haar mit Wasser gespült und sodann mit dem vorstehend beschriebenen Fixiermittel oxidativ nachbehandelt. Nach einer Einwirkungszeit von 10 Minuten werden die Wickler entfernt und die Haare, falls erforderliche, nochmals mit dem Fixiermittel behandelt und sodann mit Wasser gespült. Anschließend wird das Haar zur Frisur gelegt und getrocknet.

Bei dieser Verformungsbehandlung verbleibt kein störender Mercaptangeruch im Haar.

**Beispiel 4: Fixiermittel**

| Komponente 1: | 12,0 g | Wasserstoffperoxid |
| | 0,2 g | Natriumlaurylsulfat |
| | 0,2 g | Dinatriumhydrogenphosphat |
| | 0,1 g | Phosphorsäure, 80-prozentige wäßrige Lösung |
| | 0,1 g | Salicylsäure |
| | 83,4 g | Wasser |
| | 96,0 g | |
| Komponente 2: | 4,0 g | Ammoniumperoxodisulfat |
| Komponente 1 + 2: | 100,0 g | |

Die Komponenten 1 und 2 werden kurz vor dem Gebrauch miteinander vermischt und das so erhaltene Fixiermittel sodann mit 4 l Wasser verdünnt. Der pH-Wert dieses Fixiermittels beträgt 2,7.

Im Anschluß an eine Behandlung der Haare mit einem Verformungsmittel auf Thioglykolsäureester-Basis wird das Haar mit Wasser gespült und sodann mit dem vorstehend beschriebenen Fixiermittel unter Verwendung einer Umpumpvorrichtung 5 Minuten lang gespült. Nach Entfernung der Wickler wird das Haar mit Wasser gespült, zur Frisur gelegt und getrocknet.

**Beispiel 5: Fixiermittel**

100 g der Komponente 1 aus Beispiel 4 werden zusammen mit 6,0 g Ammoniumperoxodisulfat in 4 l Wasser gelöst.

Das so erhaltene Fixiermittel vom pH 2,5 wird in der in Beispiel 4 beschriebenen Weise zur oxidativen Nachbehandlung der Haare verwendet.

Nach Beendigung der Verformungsbehandlung trat kein unangenehmer Geruch der Haare auf.

**Vergleichsversuche**

Die Geruchsbelastung der Haare nach einer Verformungsbehandlung gemäß der vorliegenden Erfindung im Vergleich zu einer üblichen Verformungsbehandlung wurde von einer sechsköpfigen Expertengruppe beurteilt. Hierzu wurde das gewaschene Haar der Versuchspersonen zunächst auf Wickler gewickelt und mit einem Verformungsmittel, welches unmittelbar vor der Anwendung durch Vermischen der folgenden beiden Komponenten hergestellt wurde, durchfeuchtet.

**Verformungsmittel auf Thioglykolsäureester-Basis**

| Komponente 1: | 5,0 g | Harnstoff |
| | 2,0 g | Ammoniak, 25-prozentige wäßrige Lösung |
| | 0,5 g | Natriumlaurylalkoholdiglykolethersulfat |
| | 0,5 g | Ammoniumhydrogencarbonat |
| | 92,0 g | Wasser |
| | 100,0 g | |
| Komponente 2: | 30,0 g | Thioglykolsäureglycerinester |
| | 20,0 g | Glycerin |
| | 50,0 g | |

Nach einer Einwirkungszeit von 30 Minuten wurden die Haare bei einem Teil der Versuchspersonen mit einem erfindungsgemäßen Fixiermittel gemäß Beispiel 1,4 oder 5 oxidativ nachbehandelt, während bei den übrigen Versuchspersonen zur oxidativen Nachbehandlung ein Fixiermittel mit ansonsten gleicher Zusammensetzung verwendet wurde, das jedoch kein Peroxodisulfat enthielt.

Die Ergebnisse dieser Untersuchungen sind in der nachfolgenden Tabelle zusammengefaßt.

**Tabelle**

| Anzahl der Versuchspersonen | Fixiermittel nach | Anzahl der Versuchspersonen, bei denen eine Geruchsbelastung des Haares festgestellt wurde |
| --- | --- | --- |
| 20 | Beispiel 1, Komponente 1 (ohne Zusatz der Komponente 2) | 4 |
| 11 | Beispiel 1 | 0 |
| 20 | Beispiel 4, Komponente 1 (ohne Zusatz der Komponente 2) | 10 |
| 20 | Beispiel 4 | 2 |
| 13 | Beispiel 5 | 0 |

Wie die vorliegende Tabelle zeigt, wird bei Verwendung des erfindungsgemäßen Fixiermittels zur oxidativen Nachbehandlung bei der dauerhaften Haarverformung die Geruchsbelastung der Haare gegenüber üblichen Fixiermitteln erheblich vermindert.

Alle Prozentangaben in der vorliegenden Anmeldung stellen Gewichtsprozent dar.

**Patentansprüche**

1. Saures Fixiermittel für die Durchführung der oxidativen Nachbehandlung bei der dauerhaften Haarverformung auf der Basis von Wasserstoffperoxid, dadurch gekennzeichnet, daß es ein weiteres physiologisch verträgliches Oxidationsmittel, dessen Oxidationspotential größer ist als das Oxidationspotential von Wasserstoffperoxid, enthält.

2. Fixiermittel nach Anspruch 1, dadurch gekennzeichnet, daß das physiologisch verträgliche Oxidationsmittel, dessen Oxidationspotential größer ist als das Oxidationspotential von Wasserstoffperoxid, ein Alkali- oder Ammoniumperoxodisulfat ist.

3. Fixiermittel nach einem der Ansprüche 1 oder 2, dadurch gekennzeichnet, daß es 0,1 bis 18

EP 0 360 986 B1

Gewichtsprozent Wasserstoffperoxid und 0,05 bis 5 Gewichtsprozent eines weiteren physiologisch verträglichen Oxidationsmittels, dessen Oxidationspotential größer ist als das Oxidationspotential von Wasserstoffperoxid, enthält.

4. Fixiermittel nach einem der Ansprüche 1 bis 3, dadurch gekennzeichnet, daß es das Wasserstoffperoxid und das physiologisch verträgliche Oxidationsmittel, dessen Oxidationspotential größer ist als das Oxidationspotential von Wasserstoffperoxid in einem molaren Verhältnis von 1 : 0,0076 bis 1 : 0,76 enthält.

5. Fixiermittel nach einem der Ansprüche 1 bis 4, dadurch gekennzeichnet, daß es einen pH-Wert von 1,5 bis 6,9 besitzt.

6. Fixiermittel nach einem der Ansprüche 1 bis 5, dadurch gekennzeichnet, daß es in Form eines Zweikomponentenpräparates abgepackt ist und unmittelbar vor Gebrauch durch Vermischen der wasserfreien, das physiologisch verträgliche Oxidationsmittel, dessen Oxidationspotential größer ist als das Oxidationspotential von Wasserstoffperoxid, enthaltenden Komponente mit der wäßrigen, Wasserstoffperoxid enthaltenden Komponente hergestellt wird.

7. Verfahren zur dauerhaften Haarverformung, bei dem man das Haar bevor und/oder nachdem man es in die gewünschte Form bringt mit einem keratinreduzierenden Verformungsmittel behandelt, mit Wasser spült, sodann mit einem Fixiermittel oxidativ nachbehandelt, mit Wasser spült, anschließend zur Frisur legt und sodann trocknet, dadurch gekennzeichnet, daß für die oxidative Nachbehandlung ein Fixiermittel nach einem der Ansprüche 1 bis 6 verwendet wird.

**Claims**

1. Acid fixing agent based on hydrogen peroxide for the oxidative after-treatment step of a permanent hair shaping process, characterised in that it contains a further physiologically compatible oxidant, of which the oxidation potential is greater than the oxidation potential of hydrogen peroxide.

2. Fixing agent according to Claim 1, characterised in that the physiologically compatible oxidant, of which the oxidation potential is greater than the oxidation potential of hydrogen peroxide, is an alkali or ammonium peroxide disulphate.

3. Fixing agent according to either of Claims 1 or 2, characterised in that it contains 0.1 to 18 weight percent hydrogen peroxide and 0.05 to 5 weight percent of a further physiologically compatible oxidant, of which the oxidation potential is greater than the oxidation potential of hydrogen peroxide.

4. Fixing agent according to any one of Claims 1 to 3, characterised in that it contains the hydrogen peroxide and the physiologically compatible oxidant, of which the oxidation potential is greater than the oxidation potential of hydrogen peroxide, in a molar ratio of 1:0.0076 to 1:0.76.

5. Fixing agent according to any one of Claims 1 to 4, characterised in that it has a pH value of 1.5 to 6.9.

6. Fixing agent according to any one of Claims 1 to 5, characterised in that it is packed in the form of a two component preparation and is produced immediately before use by mixing the anhydrous component, containing the physiologically compatible oxidant, of which the oxidation potential is greater than the oxidation potential of hydrogen peroxide, with the aqueous component containing hydrogen peroxide.

7. Process for the permanent shaping of hair, in which the hair is treated with a keratin-reducing shaping agent before and/or after it has been arranged in the desired shape, is rinsed with water, is subsequently treated oxidatively with a fixing agent, rinsed with water, is subsequently set in a hairstyle and then dried, characterised in that a fixing agent according to any one of Claims 1 to 6 is used for the oxidative after-treatment.

**Revendications**

8

1. Fixateur acide pour effectuer le post-traitement oxydant lorsqu'on fait une permanente des cheveux à base d'eau oxygénée, caractérisé en ce qu'il contient un autre produit d'oxydation physiologiquement compatible dont le potentiel d'oxydation est supérieur au potentiel d'oxydation de l'eau oxygénée.

2. Fixateur selon la revendication 1, caractérisé en ce que le produit d'oxydation physiologiquement compatible, dont le potentiel d'oxydation est supérieur à celui de l'eau oxygénée, est un peroxodisulfate d'un métal alcalin ou d'ammonium.

3. Fixateur selon l'une des revendications 1 ou 2, caractérisé en ce qu'il contient 0,1 à 18% en poids d'eau oxygénée et 0, 05 à 5% en poids d'un autre produit d'oxydation physiologiquement compatible dont le potentiel d'oxydation est supérieur à celui de l'eau oxygénée.

4. Fixateur selon l'une des revendications 1 à 3, caractérisé en ce qu'il contient l'eau oxygénée et le produit d'oxydation physiologiquement compatible, dont le potentiel d'oxydation est supérieur à celui de l'eau oxygénée, dans un rapport molaire compris entre 1 : 0,0076 et 1 : 0,76.

5. Fixateur selon l'une des revendications 1 à 4, caractérisé en ce qu'il a une valeur de pH comprise entre 1,5 et 6,9.

6. Fixateur selon l'une des revendications 1 à 5, caractérisé en ce qu'il est emballé sous la forme d'une préparation à deux composants et qu'il est préparé immédiatement avant l'emploi en mélangeant le composant anhydre contenant le produit d'oxydation physiologiquement compatible, dont le potentiel d'oxydation est supérieur à celui de l'eau oxygénée, avec le composant aqueux contenant l'eau oxygénée.

7. Procédé pour permanenter les cheveux, selon lequel, avant et/ou après avoir donné aux cheveux la forme souhaitée, on les traite avec un produit de déformation réduisant la kératine, on les rince à l'eau, puis on effectue un post-traitement oxydant avec un fixateur, on rince à l'eau et on fait la mise en plis et on sèche, caractérisé en ce qu'on utilise pour le post-traitement oxydant un fixateur selon l'une des revendications 1 à 6.